# EUROPEAN PATENT SPECIFICATION

(11) **EP 1 410 020 B1**
(45) Date of publication and mention of the grant of the patent: **09.11.2011**
(21) Application number: 02764761.9
(22) Date of filing: 23.07.2002
(51) Int. Cl.: G01N 33/50

(54) **TEST SYSTEM AND METHOD SUITABLE FOR SELECTING TEST MATERIALS AND FORMULATIONS**
TESTSYSTEM UND VERFAHREN ZUR AUSWAHL VON MATERIALEN UND FORMULIERUNGEN
SYSTEME ET PROCEDE DE TEST DESTINES A LA SELECTION DES MATIERES DE TEST ET DE PREPARATIONS

(30) Priority: 23.07.2001 EP 01306295
(43) Date of publication of application: 21.04.2004
(73) Proprietor: PHARES PHARMACEUTICAL RESEARCH N.V., Curaçao (AN)
(72) Inventor: LEIGH, Steve, 1000 CX Amsterdam (NL); LEIGH, Mathew Louis Steven, 4052 Basel (CH); VAN HOOGEVEST, Peter, CH-4416 Bubendorf (CH); TIEMESSEN, Henricus, 79576 Weil am Rhein (DE)
(74) Representative: Hasler, Erich
(86) International application number: PCT/EP2002/008215
(87) International publication number: WO 2003/010330

(56) References cited:
- US-A- 4 927 879
- US-A- 4 931 498
- LOIDL-STAHLHOFEN A., ECKERT A., HARTMANN T AND SCHÖTTNER M: "Solid-supported lipid membrane as a tool for determination of membrane affinity: high-throughput screening of a physicochemical parameter" J. PHARM. SCIENCES, vol. 90, no. 5, May 2001 (2001-05), pages 599-606, XP002232186
- VON CORSWANT C. AND THORÉN PER E.G.: "Solubilization of sparingly soluble active compounds in Lecithin-Based microemulsions: influence on phase behavior and microstructure" LANGMUIR, vol. 15, no. 11, 1999, pages 3710-3717, XP002188168
- BETAGERI G.V. AND DIPALI S.R.: "Partitioning and thermodynamic of dipyridamole in the n-octanol/buffer and liposome systems" J. PHARM PHARMACOL, vol. 45, 1993, pages 931-933, XP009006041
- GO M-L AND NGIAM T-L: "Thermodynamics of partioning of the antimalarial drug Mefloquine in phospholipid bilayer and bulk solvents" CHEM.PHARM.BULL., vol. 45, no. 12, 1997, pages 2055-2060, XP001145780

## Description

The present invention relates generally to a method for screening materials to obtain key physicochemical and biological data. More specifically, it relates to an improved system that is suitable for testing compounds with low water solubility, particularly for compounds that are very slightly water soluble or insoluble using a novel Transmembrane screening (TS) technique for key parameters such as solubility, membrane affinity, partitioning and transfer properties in drug discovery. The invention may also be used in formulation studies to screen and select solubilising components that may improve absorption of both novel and existing compounds. The invention is highly adaptable, mimics the properties of natural bio-membranes and allows the determination of desired physico-chemical properties as well as identifying more specific information from a single screen or a series. Prior screening methods which employ membrane lipids for screening are based on either drug concentration measurements with the substrate or the surrounding aqueous phase, for determining one or other membrane property. The invention is practical, easy to perform, allows larger numbers of samples to be screened in minute amounts and thereby improve screening efficiency. The invention may also be incorporated into high throughput screening methods.

"Test system" comprises principally membrane lipid compositions in suspension or dispersion, optionally with other components for use with a set of procedure and experimental method for screening compounds.

"Activity" refers to the antagonism, agonism, inhibition, neutralisation, or other physiological effect elicited on the target or host.

" Material" includes any chemical or biological substance, any organic or inorganic element or compound, including nucleotide, single nucleotide, single nucleotide polymorphism and precursors thereof, or nucleotide sequence (including DNA and RNA sequences, gene, vector or construct including plasmids, or viruses), host organism (including fungi, algae, protozoa and hybridomas, eukaryotic or prokaryotic cell line or expression system or any development strain or product of that cell line or expression system), protein (including any peptide or amino acid sequence, enzyme, antibodies or protein conferring targeting properties and any fragment of a protein or a peptide enzyme or anti-body), cell cultures, drug or pro-drug, assay or reagent, or any genetic or biologic material, or membrane lipid component, or micro-organism or multi-cellular plants or components of living organisms. They may belong to product groups like pharmaceuticals, biopharmaceuticals, neutraceuticals, cosmeceuticals , components used in biotechnology, food components, veterinary and in-vitro and in-vivo diagnostic products. The expressions 'material', 'compound' and 'drug' are used interchangeably in this specification in order to more appropriately illustrate the specific applications.

"Test material" is the material that is screened for a desired physico-chemical

"Compound with low water solubility" covers lipophilic, hydrophobic or amphipathic compounds that require more than 10 parts of water to dissolve 1 part of the compound. It spans the definitions between sparingly soluble (from 10 to 30) to insoluble compounds (10,000 and over) but particularly very slightly soluble (from 1000 to 10'000) to insoluble (10,000 and over) as defined in USP 24.

"Transmembrane screen" (TS) describes experiments, procedures, and analytical methods for obtaining physico-chemical and biological data of a test material."

"Molecular association" between Test material and lipid molecules is achieved if not more than 10% of un-associated test material is retained on a 200 nm polycarbonate membrane filter after filtration of at least 1:5 dilution of the mixture containing the test material with distilled water.

The recent decoding of the human genome has presented the pharmaceutical industry with a wealth of opportunities to discover new protein structures related to the genome sequence. In turn these proteins serve as models to design specific molecules able to interact with the receptor proteins. At least 40 % of discovery molecules have low water solubility. The large numbers of real and virtual compounds emanating from combinatorial chemistry and computer modelling provide vast numbers of candidates for compound libraries. Screening of the more promising candidates from this library for further investigation should be rapid and predictive. Therefore it is necessary to employ efficient and inexpensive screening methods to facilitate and accelerate the drug discovery and development programs. In early testing of novel compounds for key physico-chemical properties, the limiting factors may be the absence of a fast, reliable method for predicting'good' candidates and the small amount of the compound, often less than 10mg that may be available. Although HTS (High Throughput Screen) has been made more efficient by increasing the sensitivity and speed of spectroscopic detection methods, development of robotic techniques and the use of increasingly selective receptor/substrate combinations, it is reported that 39 % of the selected drug candidates are not screened out by the screening procedures and fail during further development because of inappropriate ADME (Absorption, distribution, metabolism, elimination properties (Kennedy, T., Drug Discovery Today, 1997, 436). One reason for failure is the unreliability of current HTS methods to reliably predict 'good' and 'developable' drug candidates based on physico-chemical profiles obtained in early screens. Information on membrane interactions which may be more predictive of drug absorption is not reliable from the primary and early screens routinely used in drug discovery because of costs and time considerations. Clearly, there is a need to direct closer attention in early screening towards key membrane interactions which may be more likely to influence a favourable outcome. Mainly relying on lipophilic and solubility characteristics may not be sufficient. This is evidenced by the large number of lipophilic compounds which present absorption problems due to poor membrane affinity and transfer because they are not recognised earlier.

It is usual to carry out the characterisation of physico-chemical properties such as lipophilicity, from log octanol/water distribution considerations. The method is cumbersome and does not relate reliably lipophilic properties to drug absorption. Where additional characterisation for membrane solubility, affinity and transfer is sought, methods such as liposome chromatography, liposome partitioning, solid support lipid membranes, immobilised artificial membranes chromatography, surface plasmon resonance technology using liposome coated sensor surfaces, and assays based on micro-titer filter plates covered with lecithin have been reported. The methods may be incorporated into high throughput screens but compared to simple log octanol determinations, the screens are expensive, time consuming and require sophisticated detection and analytical equipment to determine very low concentrations of lipophilic drugs. Therefore they may be difficult to justify for early screening where high throughput, speed, efficiency and low costs are important considerations. Typical methods to screen for membrane affinity which use lipid membranes are; phospholipid with membrane proteins in IAM-HPLC (US 4,927,879 and US 4,931,498); bead based lipid systems (Loidl-Stahlofen. A et al. J. Pharma Sci. 90(5), 2001, 597-604); chromatographic system described in US-A-5,707,873; cubic phases (Engström S. et al., Eur. J. Pharm Sci. 8 (1999) 243-254; SPR biosensors (Danelian E. et al., Journal of Medicinal Chemistry, 2000, Vol. 43, No. 11, 2083-2086). In general, the prior art on lipid based systems that may be used to screen for membrane interaction of materials with low water solubility is concerned mostly with membrane affinity and not so much with transfer or permeability. Transfer in this context refers to the passive diffusion of the compound into or between membrane structures. Notably, the disclosures teach away from the use of liposomes and other model lipid particles for routine screens as the methods of preparation are thought to be inconvenient and add to costs and decrease efficiency.

Another major limitation with screening methods that may provide more useful information early on relating to membrane affinity and transfer concerns the requirement for the compound to be present in solution in the aqueous phase to partition/ transfer into the biological membrane or cell model during screening. This may be difficult because of the poor water solubility unless organic solvents are used. For compounds that are very slightly soluble or water insoluble, the concentration in the surrounding perfusion medium may not be sufficient to facilitate partitioning because of low transmembrane gradients. Therefore the small amount of drug able to be detected in the membranes and internal aqueous compartments may require extremely sensitive analytical methods. Other techniques require separation of the reactants/components after incubation to determine free drug concentrations in the external aqueous medium.

A few of the shortcomings of typical screening methods which limit their use in routine screens using artificial cells and model membranes is discussed below.

The IAM (immobilised artificial membrane) -HPLC method is characterised by the use of HPLC column material covalently coated with a phospholipid monolayer. Affinity determination of a compound for the lipid mono-layer is estimated from the retention time of the drug in solution in the mobile phase. This procedure is therefore of limited use since solvents need to be used which may be detrimental to the monolayer. Therefore, only lipophilic compounds soluble in PBS at 0.1 to 1 mg/ml or in methanol can be tested

(Stewart B.H. and Chan O.H. J. Pharm Sci. 1998 (87),12,1471-1478). Only lipophilic compounds within this solubility range can be tested. In addition, the method only provides an estimate of the affinity of the drug for the lipid mono-layer. Information on transmembrane permeability across multiple bi-layers is not obtained. In practice, the mono-layer does not mimic a natural membrane sufficiently since the latter is composed of more than a single bi-layer.

The bead-based assay method, sold under the trade mark Transil® involves multiple steps for screening compounds using a single lipid bilayer non covalently coated on a modified surface of silica or polymer beads. The beads are suspended in buffer solutions and added to different dilutions of drug in DMSO in filter well plates. After incubation, the beads are separated by fast filtration and the filtrate analysed by HPLC for free drug. The difference between the drug concentration in the reference and the filtrate gives the amount of bound material. The method does not mimic natural bi-layer membranes because the inner mono-layer which binds to the bead surface may be perturbed and comparisons with permeability across bi-layers may not be reliable. Furthermore, the aggregation state of the material inside the membrane is not known because only the filtrate is considered. Only a fraction of dissolved material will be bound to the lipid mono layer. Since the compound has poor water solubility, the small difference between two very low values gives a high margin of error. If increased amounts of drug are added to the bead suspension, precipitated drug particles are likely to be retained on the beads and may not appear in the filtrate. Alternatively, if the particle size of the precipitated drug is much smaller than the bead size, the colloidal particles may be in the filtrate and give equally misleading information. For these reasons, Transil® can only be used for lipophilic compounds with known and sufficient water solubility and may not be suitable for use in routine screening where solubility profiles and are largely undetermined and very low water solubility may prevail.

US-A-5,707,873 describes a method of assessing mixed lipid transport properties of a compound. The method provides for a mixed lipid composition which incorporates a drug as colloidal particles subjected to size exclusion chromatography. Drugs which elute with the mixed lipid particles are considered to have stable transport properties. The disclosure does relate to screening for key physico-chemical and biological properties e.g. solubility and passive transfer across membranes.

Cubic phases have also been described for measurement of drug partitioning into lipid bilayers in a HTS setting (Engström S. et al., Eur. J. Pharm Sci. 8 (1999) 243-254). However, the authors recognise that the lipid used, glyceryl mono-oleate is not a typical naturally occurring phospholipid bilayer. In this study, only affinity and not permeability is considered. The method requires separation of the components in the substrate and may not be easily adapted for screening small quantities of large numbers of compounds rapidly and effectively.

It is therefore an object of the present invention to provide a method which comprises a test system for screening of compounds to establish both overall and more specific physico-chemical parameters earlier. One of the virtues in the technique is that the measurements may be carried out in the first instance on the Test system in its entirety i.e. without separation of the components. However, if required it does not rule out the option to separate out the components after incubation to study a particular membrane interaction. Usual separation methods such as centrifugation and simple analytical filtration are normally sufficient. The invention is an improved method that is suitable for testing compounds with low water solubility, in particular compounds classed as very slightly soluble and water insoluble, using a novel Transmembrane screening (TS) technique for screening desired parameters such as solubility, membrane affinity, partitioning and transfer properties in drug discovery. It is particularly suitable for screening small quantities of large numbers of compounds rapidly and effectively. The invention may also be used in formulation studies with optionally other components to identify and select components that may improve absorption of novel and existing compounds. By using different types of membrane lipids and optionally other components to form lipid structures and by varying the test conditions more comparative information on affinity and transfer properties may be obtained from the screen and predict more closely in vivo absorption characteristics than simple log octanol/ water determinations. The invention is particularly suitable for throughput screening of novel compounds and in formulation screens to improve the delivery of existing compounds. The invention may be used for a single determination in a single container e.g. test tube or preferably in series, e.g. in well plates with multiple micro-reservoirs.

The invention is particularly suitable to screen active test materials which are very slighty water soluble (i.e. 1 in 1000 and over) for,
- membrane affinity,
- membrane permeability, transfer
- membrane lipid compatibility.

Accordingly, the method of the invention provides for a Test system comprising at least one membrane lipid component, preferably a phospholipid dispersed in an aqueous medium, optionally with other excipients. Depending on the components used, the invention may mimic many of the properties of natural membranes, offers the possibility to use asymmetric test conditions, allows rapid and efficient screening measurements with the possibility for automation. The invention compares to a practical miniature tool and allows the use of minimal amounts of test materials, typically below 100mg, possibly below 5mg quantities for each screen in single or multiple cells, in single or preferably in a series of tests. The volume in each container maybe between 10 microlitres to about 1000 microlitres or more, preferably between 50 to 500 microlitres. However, this should not be a limiting factor and depending on the compound and method of analysis smaller or larger volumes may be used. The containers for carrying out the screen may be U-V cells, test tubes, micro test tubes, nephelometer tubes, micro reservoirs such as well plates or any suitable vessel that may be used for high speed, robotic or automated handling. The test may be performed sequentially or in parallel for high throughput. Well plates are particularly preferred because of the large numbers of micro-reservoirs available for performing a series of separate tests using a single plate. The results from one well plate represent the data from a single screen and provides the desired information on key membrane interactions.

To carry out the screen for membrane affinity, a range of concentrations for the test material is dissolved in a minimum volume of one or more suitable organic solvent. Each solution is added to the container or micro-reservoirs e.g. well plate, containing a Test System. After equilibration, the well plate is examined for presence of precipitated material to determine membrane interaction by UV or any suitable analytical method. The point of inflection on a lipid/drug ratio 'vs' transmission (turbidity) plot relates to optimum membrane affinity for the particular Test system. A series of graphs from different Test systems may be drawn and the data compared. Similarly, time/transmission and membrane transfer plots may be obtained on the compound or different compounds and varying and permutating different Test systems. Furthermore, the screening or analytical conditions may be varied to obtain additional information relating more closely to specific in vivo transfer. Therefore a large number of variables may be screened with a small amount of Test material in a single well plate. Comparative data from a series of tests performed simultaneously in each plate may provide comprehensive information to characterise the test material more reliably. Turnaround time for each screen is extremely rapid.

When bilayer forming membrane lipid compositions are used to form unilamellar vesicles with particle size between 20 and 100 nm, the screening yields information on the membrane interaction with membranes comprising typical single lipid bilayer.

When bilayer forming membrane lipid compositions are used in which the lipid is dispersed in water to form multilamellar vesicles within a size range of about 50 nm to about 20 µm, the screening yields information on the membrane interaction e.g. affinity and membrane permeation of the test material through model cells with alternating lipid bi-layers.

In another variant, the invention allows high throughput screening using lipid suspensions where the internal aqueous domains comprise a medium that is different from the external medium (e.g pH, water soluble proteins). Thus, the suspension more closely mimics the situation in the stomach and duodenum where pH differences across the membrane can exist.

When aqueous micellar lipid dispersions are used which comprise mono-acyl membrane lipids or mixtures of mono and di-acyl membrane lipids in the Test system, the screening yields information on solubilisation of lipophilic materials in the intestines prior to absorption.

A further aspect of the invention relates to evaluating membrane lipid interaction in terms of aggregation state, molecular dispersion and stability of the complex formed by the Test system. The Test system disperses in water and is optically clear initially and allows reproducible transmission of light in its entirety, which can be measured using an appropriate analytical method. All that is needed after incubation is qualitative visual inspection of the turbidity of the well plate or other micro reservoirs to obtain the required information. Optionally if required, simple centrifugation or filtration may be used and quantitative analysis for more specific physico-chemical data may be made using image analysers, light microscopic observation and spectrophotometric analysis in the UV-Vis region or IR, preferably NIR region or by fluorescence as determined by the physico-chemical properties of the test material Molecular association of the active material in the Test system may be determined by means of automated sequential filtration through a 0.2 µm pores size filter plate. It should be clearly understood that the Test system in the micro-reservoirs comprising the complex formed after incubation may be introduced to biological cell models and cell lines for screening for desired biological properties. In these instances these may be regarded as alternative detection methods.

The Test system may be used to assess the membrane interaction properties of compounds with unknown solubility in initial drug discovery. The main requirement in utilising the invention is for the material to have sufficient solubility in minimum quantities of water miscible solvents with optionally small amounts of an aqueous medium and/or surfactants. Preferably the compound is in solution, but may be in suspension.

The present invention provides a reliable and reproducible method for screening for membrane lipid interactions of compounds having low water solubility by a) preparing a solution or suspension of a compound having low water solubility in at least one water miscible organic liquid with optionally small amounts of an aqueous medium and/or excipients.
b) preparing a Test system, which is optically clear and/or allows reproducible transmission of light, comprising at least one membrane lipid i) in homogeneous suspension in water or an aqueous medium ii) optionally other excipients and components
c) mixing a solution of said compound with low water solubility with at least one of said compositions with at least one membrane lipid in suspension;
and d) analysing the resultant mixture for desired physico-chemical and or biological data without further separation.

Preferably the screen is performed in e.g. 96 (or less) or 384 (or more) well plates to enable multiple testing. However, the apparatus or container used for the screen or the volumes used are not limiting factors and the invention may be incorporated into most high throughput screening procedures.

The present invention provides a Test system and the use thereof, to screen for interaction of materials with lipophilic and amphiphilic characteristics under controlled conditions such as temperature and equilibration time. The invention includes particularly the use of homogeneous suspensions and dispersions of diacyl membrane lipids, diacyl membrane lipids enriched with monoacyl membrane lipids or monoacyl lipids alone. The invention includes the use of unilamellar phospholipid vesicles suspensions to assess lipid bilayer affinity. It covers the use of multilamellar vesicles to assess both bilayer affinity and bi-layer permeability characteristics of test materials. It also includes non vesicular membrane lipid structures such as micelles, mixed micelles and microemulsions etc to assess permeability and transfer. The Test system further includes optionally, other pharmaceutically acceptable solubilising components including but not limited to e.g. cyclodextrins and derivatives thereof, polymers and surfactants.

The membrane lipid components for the Test system may be prepared as a stock dispersion. The preferred lipid type used is phosphatidylcholine (PC). Further preferred lipids and lipid mixtures include either a mixture of diacyl (PC) and monoacyl (MAPC) components in a ratio of 1:20 to 20:1, preferably 1:10 to 10:1, most preferably 1:5 to 5:1, Most preferred diacyl phosphatidylcholines are soy PC, Egg PC, POPC (1-pabnitoyl, 2-oleoylphosphatidylcholine), OOPC (1,2 dioleoylphosphatidylcholine) and partially hydrogenated Soy and Egg PC reaching a similar fatty acid composition as POPC. AIso negatively charged synthetic, semi-synthetic or naturally occurring phosphatidylserines, preferably 1,2 di-oleoylphosphatidylserine, phosphatidic acids, phosphatidylinositols, phosphatidylglycerol, preferably 1,2 dioleoylphosphatidylglycerol and 1-palinitoly,2-oleoyl phosphatidylglycerol, Egg derived PG and soy derived PG may be used. Most preferred monoacyl counterparts of the above mentioned diacyllipids include enzyme modified (Phospholipase A2) soy PC, followed by Egg PC, 1 -palmitoyl PC, 1 oleoyl PC, 1-stearoyl PC. Preferred suspensions or dispersions of membrane lipids in water contain between 0.1 to 20 % w/w preferably between 0.1% to 5% and most preferably between 0.25% to 2.0%. Unilamellar structure should preferably have a particle size smaller then about 100 nm. Multilamellar structures should preferably have a particle size ranging from about 50 nm to 20 µm with less than 30 % of the lipid located in the outer membrane of the multilamellar structure.

The Test system comprises at least one solubilising component, preferably at least one phospholipid of the formula 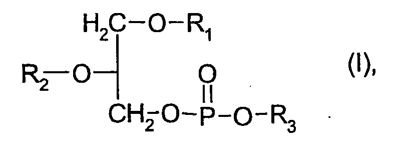 wherein
R₁ represents C₁₀-C₂₀acyl;
R₂ represents hydrogen or C₁₀-C₂₀acyl;
R₃ represents hydrogen, 2-trimethylamino-1-ethyl, 2-amino-1-ethyl, C₁-C₄alkyl, C₁-C₅alkyl substituted by carboxy, C₂-C₅akyl substituted by carboxy and hydroxy, C₂-C₅alkyl substituted by carboxy and amino, an inositol group or a glyceryl group or a salt of such compound.

Other examples of membrane lipids are cardiolipin, phosphatidylethanolamines, sphin-gomyelin, cerebrosides, glycolipids, ceramides, cholesterol and cholesterol derivatives. These membrane lipids may be purified or crude cell membrane extracts containing other membrane components derived from natural membranes and barriers like brush border membranes, asterocytes, skin cells, epithelial cells etc. natural plant or animal or microbiological sources, synthesised or partially synthesised and hydrogenated, including polyethylene glycol (PEG) derived diacyl and monoacyl equivalents. Wherever possible, the same components should be used for screening and for delivery in order to minimise the risks from changes during development. Membrane lipids such as phospholipids are widely used excipients and are preferred because of their safety and regulatory features.

Examples of suitable organic solvents to dissolve the drug include but are not limited to: methanol, ethanol, 96% ethanol, tert-butanol, absolute glycerol, propylene glycol, ethyl lactate, polyethylene glycol 300, polyethylene glycol 400, 1,3 butandiol, succinic acid diethyl ester, triethyl citrate, dibutyl sebacate, dimethyl acetamide, DMSO, DMF, glycerineformal, glycofurol (tetraglycol), isopropanol, transcutol, lactic acid butyl ester, N-methylpyrrolidone, solketol, propylene carbonate, propylene glycol diacetate, tetrahydrofurfuryl alcohol, diethylene glycol mono ethyl ether, triacetin.

Examples of additional excipients suitable for Test system compositions include but are not limited to: benzyl alcohol, benzyl benzoate, triglycerides, medium chain triglycerides like Miglyol 810™, Miglyol 812™, Miglyol 812 N™, Miglyol 829™, Miglyol 840™ Miglyol 8810™, isopropyl myristate, isopropyl palmitate, ethyl oleate, (2-octyl dodecanol), bile salts, laurinsäure hexyl ester, oleic acid, ricinus oil, sesame oil, soybean oil, salts like NaCl, CaCl₂, MgCl₂, buffer components like NaH₂PO₄, Na₂HPO₄, citric acid, HCl, NaOH, Tris, imidazole, borate, cyclodextrins, sugars like lactose, glucose, mannose, sucrose, polyalcohols like mannitol, sorbitol, , anti-oxidants like alpha tocopherol acetate, ascorbyl palmitate, antimicrobial preservatives like methyl and butyl parabene, thiomersal, sodium azide.

The Test system may be prepared as follows:

A homogeneous suspension or dispersion containing, at least one membrane lipid and optionally other excipient(s) is prepared by suspending the lipid in an aqueous medium. The aqueous medium may be buffer solutions, D2O, water and optionally may contain minor amounts of water miscible organic liquid. A suspension comprising unilamellar vesicles with diacyl PC is prepared by suspending the lipid in water followed by high shear/high pressure homogenisation until the required particle size, e.g. below 100 nm is obtained. After sterile filtration the suspension is filled into glass vials and closed with rubber stoppers and aluminium caps. Multilamellar vesicles may be prepared from lyophilisates of POPC or partially hydrogenated soy or egg PC optionally containing up to 30 weight parts OOPS (1,2 dioleoylphosphatidylserine) or other negatively charged lipid and optionally containing cholesterol lyophilised from tert-butanol according to US-A-4,370,349 by addition of a predetermined volume of saline solution. Alternatively, multilamellar liposomes may be prepared by spraying organic solution on sugar or polyalcohol crystals followed by removal of the solvent under reduced pressure and hydration of the resulting lipid containing solid matrix. Any preparation method that is suitable may be used to form vesicular or non vesicular membrane lipid structures.

The following examples illustrate the invention using test materials of well characterised, known substances having poor water solubility as surrogate compounds typically screened in discovery or in later formulation testing, to obtain physico-chemical and biological data relating to membrane affinity and solubility.

### Example 1

This example represents a novel compound that is screened for overall membrane affinity properties initially using the Test system. In this example the interaction of a lipophilic Vitronection receptor antagonist with M.W of 523 and Log P (octanol/water) 2.3, is screened using a Test system comprising membrane lipid in a 96 disposable plastic well plate (Dynatech).
a) Six Test systems are prepared as described, comprising homogeneous aqueous suspensions comprising 0.5% or 1.0 % by weight of,
   i) VP 200 (a lipid mixture comprising 98% diacyl phospholipid from soy bean phospholipid
   ii) VP 805 (blend containing 35% diacyl with 65 % mono-acyl phospholipid)
   iii) VP 814 (10% diacyl phospholipid with 90 % mono-acyl phospholipid, specially supplied by Lipoid KG, FRG).
b) A stock solution of 10mg Vitronection receptor antagonist/ml is prepared in DMSO and serial dilutions made with DMSO to concentrations of 5, 2.5, 1, 0.4, 0.2, 0.1 and 0.05mg/ml.
c) 200 µl of each of the Test system from a) is dispensed into the well plate followed by 25 µl of the drug solution from b).
d) The well plate is shaken for 20 seconds and then immediately inspected visually or turbidity assessed at 690 nm at room temperature and without further separation or filtration.

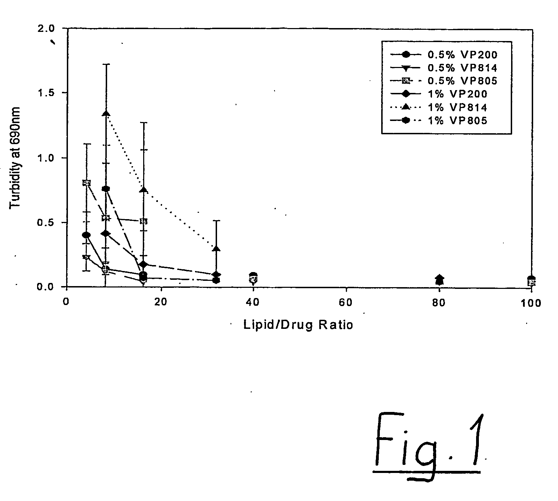

The results show that in the Test system (i) comprising 0.5 % of the compound, no increase in turbidity is observed up to 16:1 lipid/drug w/w ratio. It suggests that at this level, the drug is complexed to lipid bilayers. The affinity of the drug for bilayer forming membrane lipid prevents precipitation of the drug. Lower lipid/ drug ratios show the presence of precipitated drug. The point at which drug precipitation starts to occur quantifies the drug/membrane lipid interaction. According to this experiment mixed micelles (VP 805 and VP814) show less affinity for this drug since precipitation of the drug occurs at higher lipid/drug ratios. The results are illustrated in Fig.1.
The screen was performed in triplicate and similar results were obtained, illustrating the reproducibility of the Test system to characterise the overall membrane affinity of compounds. It should be understood that optionally, the suspensions in the well plate may be centrifuged or filtered if more detailed information on specific interaction e.g. free drug is required.

### Example 2

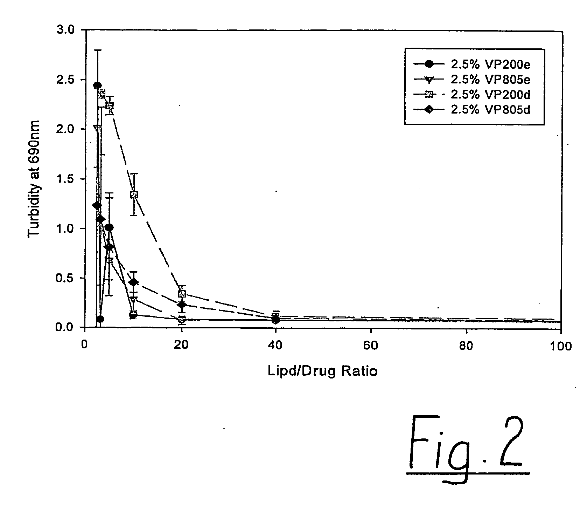

This provides a typical example of an anti-microbial agent screened for optimum activity in formulation studies.
a) A test system comprising 2.5 % VP 200 or VP 805 by weight is prepared as in example 1.
b) Solution of 80mg Glotrimazole/ml are prepared in DMSO and ethanol. Serial dilutions are made to concentrations of 60, 40, 20, 10, 5, 1, 0.4 mg/ml for both solvents.
c) 25µl of each of the solution from b) is dispensed into the 96 well plate containing 200µl of each Test system from a), as in example 1.
d) The well plate is incubated and analysed as in example 1.
After incubation, the results show that when the ethanolic Clotrimazole solutions are added to the Test systems containing VP 200 and VP 805, no change in turbidity is observed up to 20:1 lipid/ drug w/w ratio. This suggests at this lipid/ drug ratio the drug has sufficient affinity for either lipid bilayers composed of VP 200 or mixed lipid dispersions comprising VP 805 that precipitation is prevented. Lower lipid/drug ratios show the presence of precipitated drug. According to this screen, the higher turbidity of the Test stations comprising VP 805 compared to VP200 at 10:1 or lower lipid to drug ratios suggest that Clotrimazole may have higher affinity for VP 200 than VP 805. When DMSO solutions of Clotrimazole are used (VP 200d and VP 805d) the drug starts to precipitate at 20:1 lipid/drug ratio. However the higher affinity of the compound for bilayer membrane structures compared to mixed micelle structures is also evident. The results are illustrated in Fig. 2.
If further information is required on the nature of the affinity and binding characteristics from the initial coarse screen, the Test systems may be fine tuned by using different types of lipid and other components to examine the binding characteristics and transfer properties without detracting from the principle of the invention. In step c), the reservoirs used for the screen may be seeded culture plates to screen for anti-fungal properties. Alternatively in place of culture plates, Caco2 cell lines may be used to obtain biological data. In addition, the suspensions in the well plate may be centrifuged/filtered prior to incubation in cup-plates or other detection equipment to identify or compare activities.

### Example 3

This is an example of a highly lipophilic compound to assess membrane properties.
a) 0.05 % by weight of a homogeneous aqueous suspension comprising VP 200, VP 805 or VP815 is prepared.
b) A stock solution of 60 mg Cyclosporin A/ml is prepared in DMSO and ethanol. Serial dilutions are made to 40, 20, 10, 5, 2.5, 1, 0.4, 0.2, 0.1 and 0.05 mg/ml.
c) 25µl of drug solution from b) is dispensed into the 96 well plate with 200µl of lipid dispersion from a) in each micro-reservoir.
d) The well plate is incubated and analysed as in Example 1.

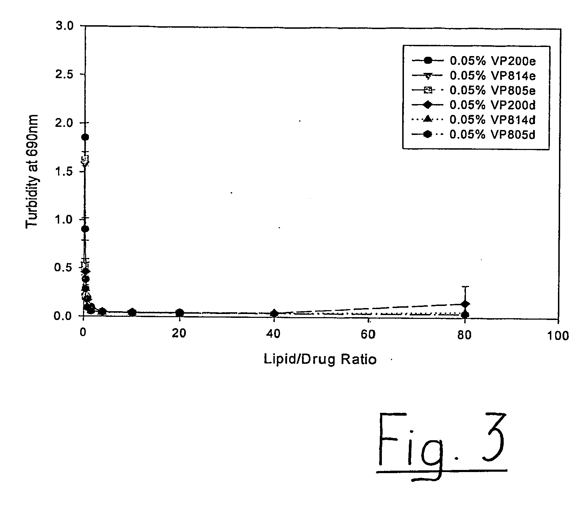

Data from the screen show that regardless of the lipid type and solvent used to dissolve cyclosporin, down to as low as 2:1 lipid/drug w/w ratio, there is no increase in the turbidity observed (Fig. 3). This means that at this ratio the amount of drug has sufficient affinity for membranes composed of VP 200 (bilayers) or VP 805 (mixed micelles) or VP 815 (micelles) to prevent precipitation. Lower lipid/ drug ratios show the presence of precipitated drug. The ratio at which drug precipitation starts to occur is therefore an indication of drug/ membrane lipid interaction. According to the screen, cyclosporin A shows a very high affinity for membrane lipids compared to the drugs tested in Example I and II. The results are depicted in Fig. 3.
Release or reverse transfer of the compound from the bound complex back into the surrounding medium may be examined by plotting a reverse time/concentration course.

The following figures illustrate the invention with reference to the above examples.
Fig.1 illustrates the influence of lipid/drug ratio on the turbidity of several drug lipid mixtures;
Fig. 2 illustrates the influence of lipid/Clotrimazole ratio on the turbidity of several drug lipid mixtures; and
Fig. 3 illustrates Influence of lipid/cyclosporin A ratio on the turbidity of several drug lipid mixtures.

The present invention provides a test system and a method for screening lipophilic and amphipathic materials with low water solubility. It generally relates to membrane lipid suspensions or dispersions employed as a test system for obtaining desired physico-chemical and biological information relating to the interaction of materials with membrane lipids in early screens in discovery. It serves as a selection method for derivatives of a test material for optimal membrane affinity and membrane permeation and thereby predict the potential for absorption in vivo. Furthermore, it may be used in later screening in formulation development, to select a carrier for a test material or derivative to enable the highest drug load. The data obtained by using the invention in primary screening provide key information on physico-chemical properties early on which may affect drug absorption and may be more predictive than log-octanol determinations. Compared to other screening methods which utilise cell models such as liposomes and cell lines, Transmembrane Screen (TS) is a versatile miniature tool that requires minimal amounts of test substances. It is adaptable, practical, cost effective and may be used routinely in high throughput screening.

## Claims

1. A method suitable for screening for membrane lipid interactions of compounds comprising:
a) preparing a solution or suspension of a test material with low water solubility in at least one water miscible organic liquid;
b) preparing a test system, which is optically clear and/or allows reproducible transmission of light, comprising at least one membrane lipid in homogeneous suspension in water or an aqueous medium;
c) mixing the solution or suspension (a) comprising said test material having low water solubility in water miscible organic liquid with the test system (b) comprising at least one membrane lipid in aqueous suspension; and
d) analysing the resultant composition comprising (a) and (b) without further separation for said membrane lipid interactions.

2. The method according to claim 1 wherein a number of solutions or suspensions in (a) are prepared, said solutions representing a range of concentrations for the test material, which solutions are added to a container or micro-reservoir for desired physicochemical and/ or biological data.

3. The method of claim 1 wherein the container(s) used for carrying out the screen may be a single container such as a test tube or in series such as in well plates with multiple micro reservoirs, U-V cells, test tubes, micro test tubes, nephelometer tubes, microreservoirs such as well plates or any suitable vessel that may be used for high speed, robotic or automated handling.

4. The method according to any one of claims 1 - 3 wherein the Test system in step b) comprises unilamellar phospholipid vesicles (SUV); multilamellar vesicles (MLV); non vesicular membrane lipid structures such as micelles, mixed micelles and microemulsions.

5. The method according to any one of claims 1-4 wherein after equilibration of the mixture of step (c) membrane lipid interactions are analysed in step (d) by examination of said mixture for presence of precipitated material whereby preferably said examination is conducted by means of qualitative visual inspection of the turbidity.

6. The method according to any one of claims 1-5 wherein said membrane lipid interactions are analysed in step (d) optionally after centrifugation or filtration at the end of the equilibration period, using simple centrifugation or filtration and quantitative analysis for more specific physico chemical data using image analysers, light microscopic observation and spectrophotometric analysis in the UV-Vis region or IR, preferably NIR region or by fluorescence, and automated sequential filtration through 0.2 µm pore size filter plate to determine molecular association of the active material in the Test system.

7. The method according to any one of claims 1-6 wherein the Test system comprising a complex formed after incubation in step (d) is introduced to biological cell models and cell lines for screening for desired biological properties as alternative detection methods.

8. The method according to any one of claims 1-6 wherein said at least one membrane lipid, being a phospholipid of the formula 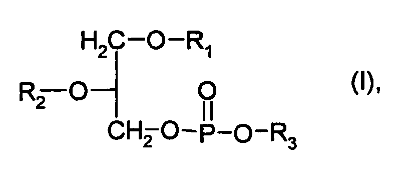 wherein
R1 represents C10-C20acyl;
R2 represents hydrogen or C10-C20acyl;
R3 represents hydrogen, 2-trimethylamino-1-ethyl, 2-amino-1-ethyl, C1-C4alkyl, C1-C5alkyl substituted by carboxy, C2-C5alkyl substituted by carboxy and hydroxy, C2-C5alkyl substituted by carboxy and amino, an inositol group or a glyceryl group or a salt of such compound.

9. The method according to any one of claims 1 -8 wherein said organic liquid in step (a) is selected from the group consisting of methanol, ethanol, 96% ethanol, tert-butanol, absolute glycerol, propylene glycol, ethyl lactate, polyethylene glycol 300, polyethylene glycol 400, 1,3 butandiol, succinic acid diethyl ester, triethyl cit rate, dibutyl sebacate, dimethyl acetamide, DMSO, DMF, glycerineformal, glycofurol (tetraglycol), isopropanol, transcutol, lactic acid butyl ester, N-methylpyrrolidone, solketol, propylene carbonate, propylene glycol diacetate, tetrahydrofurfuryl alcohol, diethylene glycol mono ethyl ether, and triacetin.

10. The method according to any one of claims 1 - 9 wherein step (b) further comprises at least one additional excipient selected from the group of excipients consisting of; benzyl alcohol, benzyl benzoate, triglycerides, medium chain triglycerides like Miglyol 810TM, Miglyol 812TM, Miglyol 812 NTM, Miglyol 829TM, Miglyol 840TM Miglyol 8810TM, isopropyl myristate, isopropyl palmitate, ethyl oleate, (2-octyl dodecanol), bile salts, laurinsäure hexyl ester, oleic acid, ricinus oil, sesame oil, soybean oil, salts like NaCI, CaCl2, MgCl2, buffer components like NaH2PO4, Na2HP04, citric acid, HC1, NaOH, Tris, imidazole, borate, solubilising components such as cyclodextrins and derivatives thereof, polymers and surfactants, sugars like lactose, glucose, mannose, sucrose, polyalcohols like mannitol, sorbitol, , anti-oxidants like alpha tocopherol acetate, ascorbyl palmi- tate, antimicrobial preservatives like methyl and butyl parabene, thiomersal, sodium azide.

11. The method according to any of the preceding claims wherein the amount of Test material prepared in step a) is typically below 100mg and below 5mg quantities for each screen in single or multiple cells, in single or preferably in a series of tests.

12. The method according to claim 11 wherein the volume in each container is between 10 µL to about 1000 µL.

13. The method according to claim 1 and claims 11 - 12 which is incorporated into high throughput screening procedures (HTS).

14. A method according to any one of claims 1 to 13 for screening and characterising active test materials which are very slightly soluble in water as defined in USP 24 for membrane lipid interactions comprising,
- (i) membrane affinity and solubility,
- (ii) membrane permeability and transfer,
- (iii) membrane lipid compatibility,
wherein said variables may be screened in single well plate or micro reservoirs, in single, or in a series of tests.

15. Use of the method according to any one of claims 1 to 14 for identifying components in
(i) formulation studies and select components that may improve absorption of novel and existing compounds,
(ii) formulation screens to improve the delivery of existing compounds in step a),
(iii) high throughput screening (HTS).

## Patentansprüche

1. Eine Methode geeignet zum Screening von Membranlipidinteraktionen von Gemischen beinhaltend:
a) Vorbereiten einer Lösung oder Suspension eines Testmaterials mit geringer Wasserlöslichkeit in wenigstens einer wassermischbaren organischen Flüssigkeit;
b) Vorbereiten eines Testsystems beinhaltend wenigstens ein Membranlipid in homogener Suspension in Wasser oder einem wässrigen Medium, welches Testsystem optisch klar ist und/oder reproduzierbare Lichttransmission zulässt;
c) Mischen der Lösung oder Suspension (a) beinhaltend das genannte Testmaterial mit geringer Wasserlöslichkeit in wassermischbarer organischer Flüssigkeit mit dem Testsystem (b) beinhaltend wenigstens ein Membranlipid in wässriger Suspension, und
d) Analysieren der resultierenden Zusammensetzung beinhaltend (a) und (b) ohne weitere Separation für die genannten Membranlipidinteraktionen.

2. Die Methode nach Anspruch 1, **dadurch gekennzeichnet, dass** eine Anzahl von Lösungen oder Suspensionen in (a) vorbereitet werden, die genannten Lösungen einen Konzentrationsbereich für das Testmaterial bilden, und die genannten Lösungen für gewünschte physiochemische und/oder biologische Daten einem Container oder Mikroreservoir zugegeben werden.

3. Die Methode nach Anspruch 1, **dadurch gekennzeichnet, dass** der oder die Container, welche zur Durchführung des Screening verwendet werden, einen einzelnen Container wie zum Beispiel ein Teströhrchen, Container in Serie wie zum Beispiel in Lochplatten mit mehreren Mikroreservoirs, UV-Zellen, Teströhrchen, Mikroteströhrchen, Nephelometerröhrchen, Mikroreservoirs wie zum Beispiel Lochplatten oder ein beliebiges geeignetes Gefäss, das für Hochgeschwindigkeitshandling, Roboterhandling oder automatisches Handling verwendet werden kann, beinhalten können.

4. Die Methode nach einem der Ansprüche 1-3, **dadurch gekennzeichnet, dass** das Testsystem in Schritt b) unilamellare Phospholipidvesikel (SUV); multilamellare Vesikel (MLV); nicht-vesikulare Membranlipidstrukturen wie zum Beispiel Mizellen, Mischmizellen und Mikroemulsionen beinhaltet.

5. Die Methode nach einem der Ansprüche 1-4, **dadurch gekennzeichnet, dass** nach Gleichgewichtseinstellung der Mischung von Schritt (c) in Schritt (d) Membranlipidinteraktionen durch Prüfung der genannten Mischung auf ein Vorhandensein von niedergeschlagenem Material analysiert werden, wobei vorzugsweise die genannte Prüfung mit Hilfe einer qualitativen visuellen Inspektion der Trübung durchgeführt wird.

6. Die Methode nach einem der Ansprüche 1-5, **dadurch gekennzeichnet, dass** die genannten Membranlipidinteraktionen in Schritt (d) wahlweise nach einer Zentrifugierung oder Filtration am Ende der Gleichgewichtseinstellungsperiode analysiert werden, und zwar unter Verwendung von einfacher Zentrifugierung oder Filtration und quantitativer Analyse für spezifischere physikochemische Daten unter Verwendung von Bildanalysesystemen, lichtmikroskopischer Beobachtung und spektroskopischer Analyse in der UV-Vis Region oder IR-Region, bevorzugt NIR-Region, oder durch Fluoreszenz, und automatisierter sequenzieller Filtration durch eine Filterplatte mit einer Porengrösse von 0,2 µm, um molekulare Assoziation vom aktiven Material im Testsystem zu bestimmen.

7. Die Methode nach einem der Ansprüche 1-6, dadurch charakterisiert, dass das Testsystem, welches einen Komplex geformt nach Inkubation in Schritt (d) beinhaltet, in biologische Zellmodelle und Zelllinien für das Screening von gewünschten biologischen Eigenschaften als alternative Detektionsmethoden eingebracht wird.

8. Die Methode nach einem der Ansprüche 1-6, **dadurch gekennzeichnet, dass** das genannte wenigstens eine Membranlipid ein Phospholipid der Formel 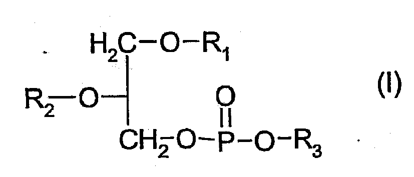 ist, worin
R₁ C₁₀-C₂₀-Acyl repräsentiert;
R₂ Wasserstoff oder C₁₀-C₂₀-Acyl repräsentiert;
R₃ Wasserstoff, zwei Trimethylamino-1-Ethyl, 2-Amino-1-Ethyl, C₁-C₄-Alkyl, C₁-C₅-Alkyl substituiert durch Carboxyl, C₂-C₅-Alkyl substituiert durch Carboxy und Hydroxy, C₂-C₅-Alkyl substituiert durch Carboxyl und Amino, eine Inositolgruppe oder eine Glycerylgruppe oder ein Salz einer solchen Verbindung.

9. Die Methode nach einem der Ansprüche 1-8, **dadurch gekennzeichnet, dass** die genannte organische Flüssigkeit in Schritt (a) ausgewählt ist aus der Gruppe bestehend aus Methanol, Ethanol, 96% Ethanol, Tert-Butanol, reinem Glycerol, Propylenglycol, Ethyllactat, Polyethylenglycol 300, Polyethylenglycol 400, 1,3-Butandiol, Succinylsäurediethylester, Treithylcitrat, Dibutylsebacat, Dimethylacetamid, DMSO, DMF, Glycerinformal, Glycofurol (Tetraglycol), Isopropanol, Transcutol, Milchsäurebutylester, N-Methylpyrrolidon, Solketol, Propylencarbonat, Propylenglycoldiacetat, Tetrahydrofurfurylalkohol, Diethylenglycolmonoethylether und Triacetin.

10. Die Methode nach einem der Ansprüche 1-9, **dadurch gekennzeichnet, dass** Schritt (b) weiter wenigstens einen zusätzlichen Hilfsstoff beinhaltet, welcher ausgewählt ist aus der Gruppe von Hilfsstoffen bestehend aus Benzylalkohol, Benzylbenzoat, Triglyceride, Medium-Chain-Triglyceride wie Miglyol 810TM, Miglyol 812TM, Miglyol 812 NTM, Miglyol 829TM, Miglyol 840TM, Miglyol 8810 TM, Isopropylmyristat, Isopropylpalmitat, Ethyloleat, (2-Octyldodecanol), Gallensäuren, Laurinsäurehexylester, Ölsäure, Rizinusöl, Sesamöl, Soyabohnenöl, Salze wie NaCl, CaCl₂, MgCl₂, Bufferkomponenten wie NaH₂PO₄, Na₂HPO₄, Zitronensäure, HCl, NaOH, TRIS, Imidazol, Borat, lösliche Komponenten wie zum Beispiel Cyclodextrine und Derivate davon, Polymere und Tenside, Zucker wie Laktose, Glukose, Mannose, Sucrose, Polyalkohole wie Mannitol, Sorbitol, Antioxidantien wie Alphatocopherolacetat, Ascorbylpalmitat, antimikrobielle Konservierungsstoffe wie Methyl- und Butylparaben, Thiomersal, Natriumazid.

11. Die Methode nach einem der vorhergehenden Ansprüche, **dadurch gekennzeichnet, dass** die Menge des Testmaterials zubereitet in Schritt (a) typischerweise unter einer Quantität von 100mg und unter einer Quantität von 5mg für jedes Screening in Einzelzellen oder multiplen Zellen, in Einzeltests oder vorzugsweise in Serientests liegt.

12. Die Methode nach Anspruch 11, **dadurch gekennzeichnet, dass** das Volumen in jedem Container zwischen 10 µL bis ungefähr 1000 µL beträgt.

13. Die Methode nach Anspruch 1 und Ansprüchen 10-12 welche in Hochdurchsatzscreening (HTS)-Abläufe eingegliedert ist.

14. Eine Methode nach einem der Ansprüche 1-13 zum Screening und Charakterisieren von aktiven Testmaterialien, welche in Wasser geringfügig löslich sind, gemäss Definition in USP 24 für Membranlipidinteraktionen beinhaltend
- (i) Membranaffinität und Löslichkeit,
- (ii) Membranpermeabilität und Transfer,
- (iii) Membranlipidkompatibilität,
worin die genannten Variablen in Einzellochplatten oder Mikroreservoirs, in Einzeltests oder in Serientests abgerastert werden können.

15. Verwendung der Methode nach einem der Ansprüche 1-14 zur Identifikation von Komponenten in
(i) Rezepturstudien und ausgewählten Komponenten, was die Absorbtion von neuen und bestehenden Gemischen verbessern kann,
(ii) Rezepturscreenings, um die Förderung von bestehenden Gemischen in Schritt (a) zu verbessern;
(iii) Hochdurchsatzscreening (HTS).

## Revendications

1. Procédé approprié pour le criblage d'interactions de lipides de membrane de composés comprenant :
a) la préparation d'une solution ou d'une suspension d'une substance d'essai avec une faible solubilité dans l'eau dans au moins un liquide organique miscible à l'eau ;
b) la préparation d'un système d'essai qui est clair optiquement et/ou qui permet la transmission reproductible de la lumière comprenant au moins un lipide de membrane dans une suspension homogène dans de l'eau ou un milieu aqueux ;
c) le mélange de la solution ou de la suspension (a) comprenant ladite substance d'essai qui a une faible solubilité dans l'eau dans un liquide organique miscible à l'eau avec le système d'essai (b) comprenant au moins un lipide de membrane dans une suspension aqueuse et
d) l'analyse de la composition résultante comprenant (a) et (b) sans autre séparation pour les interactions de lipides de membrane mentionnées.

2. Procédé selon la revendication 1 dans lequel un certain nombre de solutions ou de suspensions dans (a) sont préparées, lesdites solutions représentant un éventail de concentrations pour la substance d'essai, lesquelles solutions sont ajoutées à un conteneur ou à un microréservoir pour les données physico-chimiques et/ou biologiques souhaitées.

3. Procédé selon la revendication 1 dans lequel le(s) conteneur(s) utilisé(s) pour exécuter le criblage peuvent être un seul conteneur tel qu'un tube à essai ou une série telle que dans des plaques à puits avec des microréservoirs multiples, des cellules UV, des tubes à essais, des microtubes à essai, des tubes de néphélomètres, des microréservoirs tels que des plaques à puits ou n'importe quel récipient approprié qui peut être utilisé pour une manipulation rapide, par un robot ou automatisée.

4. Procédé selon l'une quelconque des revendications 1 à 3 dans lequel le système d'essai dans l'étape b) comprend des vésicules unilamellaires phospholipidiques (SUV), des vésicules multilamellaires (MLV), des structures de lipides de membrane non vésiculaires telles que des micelles, des micelles mélangées et des microémulsions.

5. Procédé selon l'une quelconque des revendications 1 à 4 dans lequel des interactions de lipides de membrane sont analysées dans l'étape (d) après équilibrage du mélange de l'étape (c) par l'examen dudit mélange pour la présence de substance précipitée, ledit examen étant effectué de préférence au moyen d'une inspection visuelle qualitative de la turbidité.

6. Procédé selon l'une quelconque des revendications 1 à 5 dans lequel des interactions de lipides de membrane sont analysées dans l'étape (d) en option après centrifugation ou filtration à la fin de la période d'équilibrage en utilisant la centrifugation simple ou la filtration et l'analyse quantitative pour des données physico-chimiques plus spécifiques en utilisant des analyseurs d'images, l'observation microscopique en lumière et l'analyse spectrophotométrique dans la région UV-visible ou IR, de préférence la région NIR ou par fluorescence, et la filtration séquentielle automatisée par plaque filtrante de 0,2 µm de taille de pore pour déterminer l'association moléculaire de la substance active dans le système d'essai.

7. Procédé selon l'une quelconque des revendications 1 à 6 dans lequel le système d'essai comprenant un complexe formé après incubation dans l'étape (d) est introduit dans des modèles de cellules biologiques et des lignées de cellules pour le criblage pour les propriétés biologiques souhaitées comme procédés de détection alternatifs.

8. Procédé selon l'une quelconque des revendications 1 à 6 dans lequel ledit lipide de membrane qui existe au moins étant un phospholipide de la formule 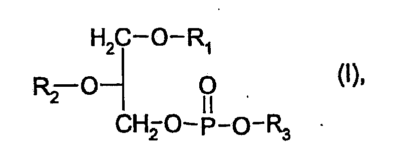 dans laquelle
R1 représente un acyle en C10-C20 ;
R2 représente l'hydrogène ou un acyle en C10-C20 ;
R3 représente l'hydrogène, le 2-triméthylamino-1-éthyle, le 2-amino-1-éthyle, un alkyle en C1-C4, un alkyle en C1-C5 substitué par un groupe carboxyle, un alkyle en C2-C5 substitué par un groupe carboxyle et hydroxyle, un alkyle en C2-C5 substitué par un groupe carboxyle et amino, un groupement d'inositol ou un groupement de glycérile ou un sel d'un tel composé.

9. Procédé selon l'une des revendications 1 à 8 dans lequel ledit liquide organique dans l'étape (a) est sélectionné dans le groupe comprenant le méthanol, l'éthanol, l'éthanol à 96%, le tert-butanol le glycérol absolu, le propylène glycol, le lactate d'éthyle, le polyéthylène glycol 300, le polyéthylène glycol 400, le 1,3 butanediol, le diéthylester d'acide succinique, le triéthylcitrate, le sébacate de dibutyle, le diméthylacétamide, le DMSO, le DMF, le glycérineformal, le glycofurol (tétraglycol), l'isopropanol, le transcutol, l'ester butylique d'acide lactique, le N-méthylpyrrolidone, le solketol, le carbonate de propylène, le diacétate de propylène glycol, l'alcool tétrahydrofurfuryl, l'éther mono-éthylique de diéthylène glycol et la triacétine.

10. Procédé selon l'une quelconque des revendications 1 à 9 dans lequel l'étape (b) comprend de plus un excipient supplémentaire sélectionné dans le groupe d'excipients comprenant : l'alcool benzilique, le benzoate de benzile, les triglycérides, les triglycérides à chaîne moynne comme Miglyol 810TM, Migylol 812TM, Miglyol 812 NTM, Miglyol 829TM, Miglyol 840TM, Miglyol 8810TM, le myristate d'isopropyle, le palmitate d'isopropyle, l'oléate d'éthyle (2-octyle dodécanol), les sels biliaires, le laurate d'hexyl ester, l'acide oléique, l'huile de ricin, l'huile de sésame, l'huile de soja, les sels comme le NaCl, le CaC12, le MgC12, les substances tampons comme le NaH2PO4, le Na2HP04, l'acide citrique, le HC1, le NaOH, le tris imidazole, le borate, les composés solubilisants tels que les cyclodextrines et les dérivés de ceux-ci, les polymères et les surfactants, les sucres tels que le lactose, le glucose, le mannose, le sucrose, les polyalcools tels que le mannitol, le sorbitol, les antioxydants comme l'alphatocophérol acétate, le palmitate d'ascorbyle, les agents de conservation antimicrobiens comme le parabène de méthyle et de butyle, le thiomersal, l'azoture de sodium.

11. Procédé selon l'une des revendications précédentes dans lequel la quantité de substance d'essai préparée dans l'étape a) est de manière typique en dessous de 100 mg et en dessous de quantités de 5 mg pour chaque criblage dans des cellules individuelles ou multiples, dans un essai unique ou de préférence dans une série d'essais.

12. Procédé selon la revendication 11 dans lequel le volume dans chaque conteneur est entre 10 µL et environ 1000 µL.

13. Procédé selon la revendication 1 et les revendications 11 et 12 qui est incorporé dans les procédures de criblage à haut débit (HTS).

14. Procédé selon l'une quelconque des revendications 1 à 13 pour le criblage et la caractérisation de substances d'essai actives qui sont très légèrement solubles à l'eau comme défini dans l'USP 24 pour des interactions de lipides à membrane comprenant
- (i) affinité et solubilité de la membrane
- (ii) perméabilité de la membrane et transfert
- (iii) compatibilité des lipides de la membrane
dans lequel lesdites variables peuvent être criblées dans une seule plaque à puits ou dans des microréservoirs dans un seul essai ou dans une série d'essais.

15. Utilisation du procédé selon l'une quelconque des revendications 1 à 14 pour identifier des composés dans
(i) des études de formulations et sélectionner des composants qui peuvent améliorer l'absorption de composés nouveaux et existants,
(ii) des cribles de formulation pour améliorer la livraison de composés existants dans l'étape (a),
(iii) le criblage à haut débit (HTS).
